Europäisches Patentamt

European Patent Office

Office européen des brevets

(11) Publication number:

**0 203 394**
A2

(12) **EUROPEAN PATENT APPLICATION**

(21) Application number: **86105843.6**

(51) Int. Cl.⁴: **H 04 M 19/04**

(22) Date of filing: **28.04.86**

(30) Priority: **29.04.85 CN 85103135**

(43) Date of publication of application: **03.12.86**
**Bulletin 86/49**

(84) Designated Contracting States: **BE DE FR GB**

(71) Applicant: **Liu, Zhong Du, No. 5, Xinjiekouwai Street, Beijing (CN)**
Applicant: **BEIJING EVER BRIGHT INDUSTRIAL CO., Room 605, Bldg no. 6 Xiyuan Hotel, Beijing (CN)**

(72) Inventor: **Liu, Zhongdu, No. 5, XinJieKouWai Street, Beijing (CN)**
Inventor: **Liu, Zhonxian, No. 5, XinJieKouWai Street, Beijing (CN)**

(74) Representative: **Ebbinghaus, Dieter et al, v. FÜNER, EBBINGHAUS, FINCK Patentanwälte European Patent Attorneys Mariahilfplatz 2 & 3, D-8000 München 90 (DE)**

(54) **Electronic music/voice ringer circuit for telephone.**

(57) An electronic music/voice ringer circuit for use in telephone sets. It can be used in telephone lines of any system and incorporated in telephone sets of any type. It consists mainly of a transistor acting as an amplifier, and an integrated music/voice signal generator which can be connected directly to ringing pulse line to convert a series of intermittent ringing pulses into continuous sweet music calling signals or voice calling signals.

0203394

LIU ZHONG DU and
BEIJING EVER BRIGHT INDUSTRIAL CO.

EPAB-33819.2
April 28, 1986

# Electronic Music/voice Ringer Circuit for Telephone

## Technical Field

The present invention relates to an electronic muic/voice ringer circuit for use in telephone sets and more particularly to a music/voice ringer circuit for use in telephone lines of any system and telephone subscriber sets of any type.

## Background of the Invention

Most telephone ringers used to be electro mechanical ringers which have the effects of higher noise and smaller audible range. A dual tone multifrequency (DTMF) telephone ringer has been introduced in recent years. U.S. Patent No. 4,309,573 discloses a loudspeaking substation circuit employing a DTMF signal generator. U.S. Patent No.4,309,574 discloses an electronic tone ringer. These types of circuits can only generate intermittent single tone or dual tone alert signals in response to ringing signals.

## Summary of the Invention

The object of the present invention is to provide a new electronic music/voice ringer circuit for use in telephone lines of any system and telephone subscriber sets of any type.

The electronic music/voice ringer ciruit according to the present invention can be incorporated into telephone subscriber sets of any type and driven by low frequency ringing pulses from a conventional switch board to generate an intermittent flashing signals and an incessant audible music/voice calling signals. It generates flashing signals first in response to incoming ringing pulses, and then generates audible music/voice calling signals if the flashing signals do not arouse subscriber's attention.

The electronic music/voice ringer circuit according to the present invention can generate a sweet melodic, trilling music calling signals or voice calling signals simulating speech sounds, which are not piercing and noisy, and audible in a larger range.

The electronic music/voice ringer circuit according to the present invention can maitain a non-trilling music calling signal different from the trilling signal for 5-15 seconds affter the ringing pulses end so as to tell the telephone subscriber that there was a incoming call just a moment ago.

The electronic music/voice ringer circuit according to the present invention is simple in structure, and it can sound continuous, sweet electronic muisc call, thereby eliminating the noises of electro mechanical ringer. In addition, this circuit is compatible with telephone lines of any system and can be used directly in telephnoe sets of any type.

Brief Description of Drawings

Fig.1 is a block diagram of the electronic music/voice ringer circuit according to the present invention;

Fig.2 shows an arrangement of the electronic music/voice ringer circuit according to the present invention;

Fig.3 shows circuit arrangement of $IC_1$.

**Description of Preferred Embodiment**

The electronic music ringer circuit according to present invention comprises a single transistor and an integrated music signal generator and a plurality of diodes, resistors and capacitors wherein the above transistor, diodes and resistor may be integrated as a micro module.

The circuit comprises a master trigger circuit (1), a power supply ciruit (2), a first trigger circuit (4), a second trigger circuit (3), a music signal amplifier (5), a trilling circuit (6), a music signal generator (8), a tone control ciruit (7) and a music signal output circuit (9). The power supply (2) is connected directly to ringing pulse circuit via a resistor-capacitor network.

Incoming ringing pulses are rectified to DC voltages and then the rectified signals are supplied to music signal amplifier (5) and music signal generator (8) affter being limited and filtered. The master trigger circuit and neon lamp (1) is triggered by a ringing pulse. As a result, a jumping trigger singal is formed While generating a jumping signal. The jumping signal starts a music program in the music signal generator (8) via the first trigger circuit (4) and sets off the operation of music signal amplifier (5) via the second trigger circuit (3).

Music signal amplifier (5) is either an audio-frequency amplifier or an electronic switch. It is in cut-off state and accordingly cuts off the operating voltage of music signal generator (8) when ringing pulses are absent. The muster trigger circuit triggered by incoming ringing pulses to generate a jumping pulse which turns on music signal amplifier (5) via second trigger circuit (3). At the same time, the music signal amplifier (5) provides operating voltage for the music signal generator (8) which is then turned on by jumping pulse via first trigger circuit (4). The music signals from music signal generator (8) are sent out by a music signal output circuit (9) after being amplified by amplifier (5) which concurrently acts as a constant current source to stabilize the suystem.

The closed loop formed by music signal amplifier (5) and music signal generator (8) continuously operates with the energy stored in capacitor $c_3$ in the intervals of ringing pulses to generate continuous music signal.

In order to improve acoustic effect a trilling circuit (6) is added to amplifier (5) to produce low-frequency mudulated music, hence to produce special trill which makes music sound sweet and intersting.

- 5 -

Furthermore, a tone control circuit (7) is provided according to the present invention, which can change the main oscilatring frequency of the music signal generator (8) to change the tone of music. Thus , several telephone sets in a room with different tones of music can be distinguished from one another.

The off-hook switch of a telephone set can cutoff the music signal generator and the amplifier.

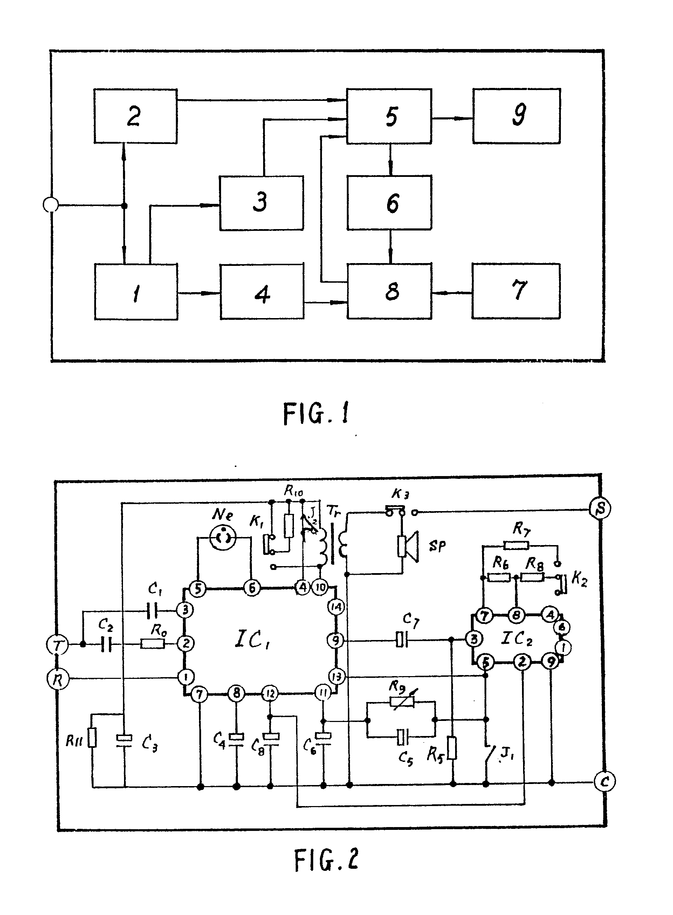

The electronic voice ringer circuit according to the present invention may employ an integrated voice synthesis circuit which can simulates speech sounds instead of music signal generator $IC_2$ in Fig 2.

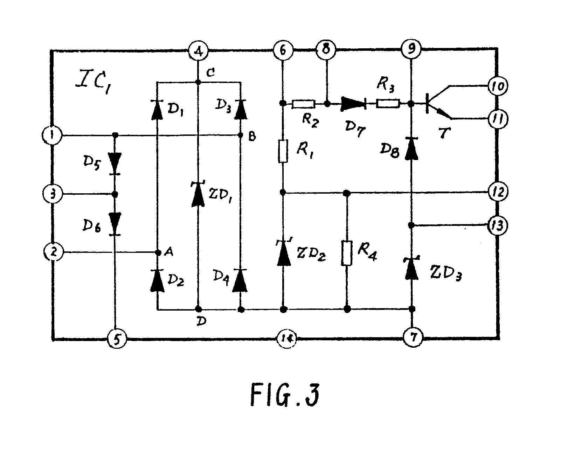

Referring now to Fig.2 and 3. Ringing pulses are fed to AC terminals A and B of the diode bridge comprising diodes $D_1$, $D_2$, $D_3$ and $D_4$ via a resistor-capacitor network composed of resistor $R_0$ and capacitor $C_2$. A Zener diode $ZD_1$ is connected across the DC output terminals C and D of the bridge to perform voltage regulation and protection. Wherein D is a common earth potential point while c is a positive DC voltage output terminal. A capacitor $C_3$ is connected between C and D to perform filtering and energy storing. The positive DC voltage from ferminal C is supplied to transistor T via primary winding of an output transformer Tr which is connected in series with the collector circuit of the transistor T. $R_{11}$ is a discharging resistor of $C_3$.

The master trigger circuit comprises copacitor $C_1$,

diodes $D_5$, $D_6$ and neon lamp Ne. Capacitor $C_1$ and diode $D_5$ in series are connected in parallel with ringing pulse line. One terminal of neon lamp is connected to the negtive end of diode $D_6$ whose positive end is connectod to conjumction of $C_1$ and $D_5$. The other terminal of neon lamp is connected to first trigger circuit and second trigger circuit. A ringing pulse is rectified by a rectifer doubler composed of capacitor $C_1$, diodes $D_5$, $D_6$. The output from negtive end of diode $D_6$ is a positive pulse whose amplitude is twice as high as the positive amplitude of the ringing pulse. The positive pulse then turns on the negtive resistonce device Ne. which gererates flashing signals. On another terminal of Neon lamp, a positive jumping pulses is formed. The second trigger circuit consists of resistors $R_2$, $R_3$, capacitor $C_4$ and diode $D_7$. The positive jumping pulse is fed to the base of trnsistor T of music signal amplifier (5) from the conjunction of resistors $R_1$ and $R_2$ through resistor $R_2$, diode $D_7$ and resistor $R_3$. The amplifier composed of transistor T is a class C amplifier which is usually in cut-off state. When positive jumping pulse provides positive bias for the base of transistor T, the transistor turns on, providing power supply for music signal generator (8). Resistor $R_2$ and capacitor $C_4$ consititute a decoupling circuit to suppress the noises of amplifier due to the noises in the ringing pulses. Zener diode $ZD_2$, resistors $R_1$, $R_4$ and capacitor $C_8$ constitute first

trigger (4). The positive jumping pulse directly initiates music signal generator (8) to start music program after being divided by resistor $R_1$ and $R_4$. Zener diode $ZD_2$ is provided to limit the amplitude of trigger pulse. Resistor $R_1$ and capacitor $C_8$ is an integrating circuit which performs antijamming. The time lag between the generation of flashing signals and that of music signals upon the comming of ringing pulses can be changed by adjusting resistance of resistor $R_1$ or capacitance of capacitor $C_8$. The music program is started by music signal generator (8), from which the output music signals are coupled to the base of transistor T, and supplied from collector to transformer Tr after being amplified.

The transformer Tr is provided to match for impednce-matching and transfering power, its secondary winding drives speaker to sound music call.

In the intervals of ringing pulses, the energy stored in filterring capacitor $C_3$ of power supply circuit (2) is supplied via transformer Tr and transistor T to $IC_2$ of music signal gencerator (8) the supply voltage to $IC_2$ is clamped to a propriate and stoble voltage by Zener diode $ZD_3$. The output of $IC_2$ is coupled to the base of transistor T via capacitor $C_7$ to contiuously turn on T. A capacitor $C_6$ is connected across the emitter of transistor T and the common earth, the emither of transistor T becoming a.c common earth, and transitor T operating in commen-emitter state. The music signals are sent into spea ker sp via transformer Tr to soccunal music signals continaousely. Resistor $R_{10}$ connected to primary winding of transformer Tr can be controlled by a switch $k_1$. When $k_1$ is open, it does not function. When $k_1$ is closed, it connects in parallel with primary winding of transformer Tr and shunts a part of music signals from collector of transistor T to supply terminal thereby to reduce output volume.

Tone control circuit consists of resistors $R^6$, $R_7$, $R_8$ and switch $k_2$. The main oscilating frequency of music signal generator can be charged by means of $k_2$, hence, the beat and tone of the music can be changed. Rosistor $R_9$ and capacitor $C_5$ in parallel are connected to the emitter of transistor T which produce a low frequency modulated oscilation together with transistor T and capacitor $C_6$ to present trilling effect and make the music more sweet and pleasant. The frequency and amplitude of trilling modulation can be changed by adjusting the resistance of resistor $R_9$.

When the telephone reciever of the subscriber set is taken up for calling, the off-hook saith $T_1$ connected between supply terminal of music signal generator (8) and the common terminal is closed to short circuit supply terminal and stop music program. Switch $J_2$ is connected with one end to positive supply terminal of the $IC_1$ and with the other end to the tramsformer Tr. When the telephone set is off hook, switch $J_2$ is open, and switch $J_1$ is closed, the music is stopped. Besides, when the telephone set is off hook, $J_2$ is open, so that the voice signals will not be shunted by capacitor $C_3$.

The electronic voice ringer circuit according to the present invention has approximately the same structure as music ringer circuit exept that the above music generator $IC_2$ is replaced by an intergrated voice syunthesis circuit $IC_2$ with the above trilling circuit omitted.

Claims

1. An electronic music ringer circuit for use in telephone lines of any system and telephone subscriber's sets of any type being characterized by comprising a power supply circuit (2) connected to telephone lines; a master trigger circuit means (1) connected to telephone lines for converting a ringing pulse into a jumping pulse; a music signal generating means (8) with a ROM in which a music program is prestored; a music signal amplifying means (5) composed of a transistor; a first trigger circuit means (4) starting the music program stored in said music signal generating means; a second trigger circuit means (3) turning on said music signal amplifying means; a trilling circuit (6); a tone control means (7) and a music calling signal output means (9).

2. An electronic voice ringer circuit for use in telephone lines of any system and telephone subscriber's sets of any type being cheracterized by comprising a power supply circuit (2) connected to telephone lines; a master trigger circuit means (1) connected directly to telephone lines for converting a ringing pulse into a jumping pulse; a voice synthesising means pre-storing a voice program; a voice signal amplifying means (5) composed of a transistor; a first trigger means (4) for starting the voice program stored in said voice symthesising means; a second trigger means (3) for turning on said voice signal amplifying means; a tone control means (7) and a voice calling signal output mean (9).

3. An electronic music ringer circuit according to claim 1 being characterized in that the a.c. input of said power supply circuit (2) is connected to the telephone line via resistor-capacitor network consisted of $R_o$ and $C_2$ in series, and a Zener diode $ZD_1$ and a capucitor $C_3$ for storing energy and filtering are connected across the D.C outputs of the rectifier bridge

4. An electronic music ringer circuit according to claim 1 Characterized in that said master trigger circuit means (1) consists of capacitor $C_1$, diode $D_5$, $D_6$ which form a rectifier **doubler** connected in series with a neon lamp which can generate flashing signals.

5. An electronic music ringer circuit according to claim 1 being characterized in that said second trigger circuit means (3) consists of resistor $R_2$, $R_3$, capacitor $C_4$ and diode $D_7$, the conjunction of $R_1$ and $R_2$ is the input of said second trigger circuit and is connected with the output of neon lamp; $R_2$ and $R_7$ are connected in series with $R_3$ from which the trigger signal is output, and $C_4$ is connected between the conjunction of $R_2$ and $D_7$ and common earth.

6. An electronic music ringer circuit according to claim 1 being characterized in that said first trigger circuit means consists of a voltage divider composed of resistors $R_1$ and $R_4$, a zener diode

$ZD_2$ and a copacitor $C_8$ connected in perallel with resistor $R_4$; trigger signal is output from $C_8$.

7. An electronic music ringer circuit according to claim 1 being characterized in that said music signal amplifying means consists of a single transistor and acts concurrently as an electronic switch and a constant current source, which is an audio frequency amplifier operable to produce trill effects in cooperation with said trilling circuit (6); the base of said transistor is connected with the output of said second trigger circuit means; the emitter of which is connected to said trilling circuit; the collector of which outputs amplified music signals to said output circuit (9).

8. An electronic music ringer circuit according to claim 1 being characterized in that said music signal generating means consists of a CMOS integrated circuit prestoring a music program, the triggerring terminal of which is connected to the output of said first trigger circuit means, the clock signal input of which is connected to said tone control circuit composed of resistors $R_6$, $R_7$, $R_8$ and switch $K_2$.

9. An electronic music ringer circuit according to claim 1 being characterized in that said trilling circuit is composed of a variable resistor $R_9$ and a capacitor $C_5$ connected in parallel with each other, one terminal of which is connected to the emitter of transistor T, the other terminal of which is connected to the supply terminal of said music signal generating means.

10. An electronic music ringer circuit according to claim 1,7,9 being characterized in that the emitter of transistor T acting as a constant current source is connected to supply terminal of said music signal generating means $IC_2$, a Zener diode $ZD_3$ performing clamping and regulating is connected between said supply terminal and common earth, an audio by-pass capacitor $C_6$ is connected between collector of transistor T and common earth.

11. An electronic music ringer circuit according to claim 1, being characterized in that a normally-closed switch $J_2$ is connected to a positive supply terminal C and synchronizes with off-hook to stop music call.

12. An electronic music ringer circuit according to claim 1, being characterized in that a normally-open switch $J_1$ is provided which is connected in paralled with supply terminal of $IC_2$ and synchronizes with off-hook to stop masic call.

13. An electronic voice rigner circuit according to claim 2, being characterized in that the a.c. input of said power supply circuit (2) is connected to the telephone line via resistor-capacitor network consisted of $R_0$ and $C_2$ in series, a Zener diode $ZD_1$ and a capacitor $C_3$ for storing energy and filtering are connected across the D.C. outputs of rectifier bridge.

14. An electronic voice ringer circuit according to claim 2 being characterized in that said master trigger circuit means (1) consists of capacitor $C_1$, diode $D_5$, $D_6$ which form a rectifier doubler connected in series with a neon lamp which can generate tlashing signals.

15. An electronic voice ringer circuit according to claim 2 being characterized in that said second trigger circuit mean (3) consists of resistor $R_2$, $R_3$, capacitor $C_4$, the conjunction of $R_1$ and $R_2$ is the input of said second trigger circuit and is connected with the output of neon lamp, $R_2$ and $D_7$ is in series with $R_3$ from which the trigger signal is output, and $C_4$ is connected between conjunction of $R_2$ and $D_7$ and the common earth.

16. An electronic voice ringer circuit according to claim 2 being characterized in that said first trigger circuit means consists of a voltage divider composed of resistor $R_1$ and $R_4$, a Zener diode $ZD_2$ and a capacitor $C_8$ connected in parallel with resistor $R_4$; trigger signal is output from $C_8$.

17. An electronic voice ringer circuit according to claim 2 being characterized in that said voice amplifying means consists of a single transistor and acts concurrently as an electronic switch and a constant current source, which is a audio frequency amplifier, the base of said transistor is connected to the output of said second trigger circuit means, the emitter of which is connected direcfly to supply terminal of said voice synthesis means; the collector of which outputs amplified voice signals to said output circuit (9).

- 15 -                                    0203394

18. An electronic voice ringer circuit according to claim 1 being characterized in that said voice synthesis means (8) consists of a integiated circuit which can simulates speech sounds, the triggerring terminal of which is connected to the output of said first trigger circuit means; the clock signal input of which is connected to said tone control means (7) composed of resistor $R_6$, $R_7$ and switch $K_2$.

19. An electronic voice rigner circuit according to claim 2,17, characterized in that the emitter of transistor T acting as a constant source is connected to supply terminal of said voice synthesis means, a Zener diode $ZD_3$ performing clamper and regulating is connected between said supply terminal and common earth; an audio by-pass capacitor $C_6$ is connected between collector of transistor T and common earth.

20. An electronic voice ringer circuit according to claim 2, being characterized in that a normally-closed switch $J_2$ is provided which is connected to positive supply terminal C and synchronizes with off-hook to stop music call.

21. An electronic voice ringer circuit according to claim 2, being characterized in that a normally-open switch $J_1$ is provided which is connected in parallel with supply terminal of $IC_2$ and synchronizes with off-hook to stop music call.

FIG. 1

FIG.2

FIG.3